# FASCICULE DE BREVET EUROPEEN

(11) **EP 0 637 712 B1**
(45) Date de publication et mention de la délivrance du brevet: **09.07.1997**
(21) Numéro de dépôt: 94401801.9
(22) Date de dépôt: 04.08.1994
(51) Int. Cl.: F16K 27/00, A61M 5/14

(54) **Rampe de robinets**
Ventilverteiler
Valve distributor

(30) Priorité: 06.08.1993 FR 9309721
(43) Date de publication de la demande: 08.02.1995
(73) Titulaire: VYGON, F-95440 Ecouen (FR)
(72) Inventeur: Rossi, Daniel, F-95630 Meriel (FR)
(74) Mandataire: Schrimpf, Robert

(56) Documents cités:
- DE-U- 8 907 824
- FR-A- 2 145 858
- FR-A- 2 267 009
- FR-A- 2 358 601
- US-A- 4 483 365

## Description

L'invention concerne les rampes de robinets utilisées notamment dans le domaine médical pour distribuer à la demande un liquide dans une ou plusieurs dérivations, chaque dérivation étant placée sous le contrôle d'un robinet.

Une rampe de robinets est connue en tant que telle du document DE-U-8 907 824.

Le but de la présente invention est de faciliter la fabrication des rampes et leur utilisation, notamment en permettant de faire varier à volonté le nombre de dérivations désirées.

On y parvient selon l'invention en constituant la rampe au moyen de plaquettes et de robinets, chaque plaquette étant munie sur deux bords opposés de moyens de solidarisation permettant de fixer les plaquettes bout à bout et chaque plaquette portant sur au moins une face un ou plusieurs robinets ou étant munie sur cette face de moyens pour le montage d'un ou de plusieurs robinets, et chaque robinet comportant un boisseau dans lequel débouchent au moins trois embouts solidaires dudit boisseau, deux des embouts, respectivement mâle et femelle, étant opposés et alignés selon un axe qui est perpendiculaire aux dits bords opposés de la plaquette lorsque le robinet est fixé sur la plaquette, et le troisième embout étant disposé obliquement ou perpendiculairement par rapport aux deux embouts alignés, ledit robinet étant conçu pour commander une communication de fluide entre les embouts alignés et le troisième embout et lesdits embouts alignés étant conçus pour que ces embouts mâles et femelles s'emboîtent de proche en proche lorsque les plaquettes qui portent les robinets sont solidarisées bout à bout.

On peut ainsi réaliser une rampe à nombre de voies quelconque, en usine ou à l'hôpital. On peut même partir d'une rampe à x-voies réalisée en usine et y rajouter, à l'hôpital, y plaquettes à robinet pour obtenir une rampe à x + y voies.

Du point de vue industriel, l'invention permet de limiter les investissements au minimum et d'obtenir une grande flexibilité commerciale, en s'adaptant aisément aux variations de la demande du marché.

Les robinets peuvent être fixés les uns aux autres par leurs embouts mâles et femelles, par exemple par collage, soudure, vissage ou encliquetage. Dans le cas du vissage, les embouts alignés d'un robinet comportent respectivement une extrémité filetée extérieurement et un écrou mobile, en sorte que l'embout à écrou du robinet d'une plaquette puisse être fixé à l'embout fileté du robinet d'une plaquette contiguë par vissage de l'écrou sur l'extrémité fileté.

Ce dernier cas correspond mieux au montage à l'hôpital, que ce soit pour constituer une rampe ex- nihilo ou pour rajouter des voies supplémentaires à une rampe réalisée en usine.

On décrira ci-après des exemples de constitution de rampes selon l'invention, en référence aux vues en perspectives du dessin joint, sur lequel :
- la fig. 1 représente une plaquette portant un robinet, constituant avec lui un module de rampe ;
- la fig. 2 représente le module de la fig. 1, tourné en sens opposé ;
- la fig. 3 représente une rampe constituée par assemblage de modules du type de la fig. 1 ;
- la fig. 4 représente une variante de module ;
- la fig. 5 représente le module de la fig. 4, tourné en sens opposé ;
- la fig. 6 représente une rampe constituée par assemblage de modules du type de la fig. 4 ;
- la fig. 7 représente une variante de rampe ;
- la fig. 8 représente deux modules selon une variante de réalisation de l'invention, vus de dessus en perspective ;
- la fig. 9 représente les deux modules de la fig. 8, vus de dessous en perspective, et
- la fig. 10 est une vue comparable à celle de la fig. 9, les modules étant vus en sens opposés.

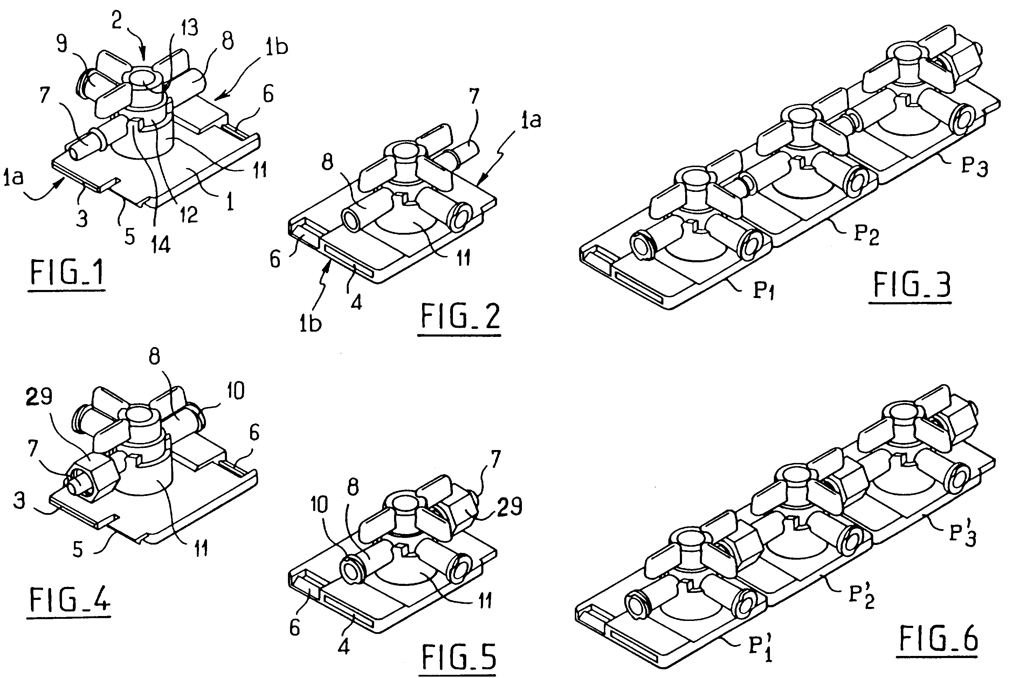

Le module représenté sur la figure 1 est constitué par une plaquette 1, en matériau de synthèse, qui porte un robinet 2, également en matériau de synthèse.

Sur chacun de deux bords opposés 1a, 1b, la plaquette est agencée pour être solidarisée par emboîtage et encliquetage avec la plaquette homologue d'un module contigu placé en amont ou en aval.

Dans cet exemple, l'un des bords la présente un tenon 3 et le bord opposé 1b présente une mortaise correspondante. En outre, l'un des bords 1a présente une languette terminée par un crampon 5 et l'autre bord 1b présente une languette terminée par un crampon 6, ces deux crampons étant tournés l'un vers le bas et l'autre vers le haut. Les crampons de deux plaquettes contiguês coopèrent par le fait que les deux languettes qui les portent ont une certaine capacité de débattement dans le plan vertical.

On comprendra que ces moyens de solidarisation ne sont indiqués qu'à titre d'exemples préférés.

Le robinet est muni de deux embouts 7,8 alignés selon un axe parallèle à la plaquette et perpendiculaire aux bords d'encliquetage 1a, 1b et un troisième embout 9 disposé perpendiculairement ou obliquement par rapport aux embouts alignés.

L'embout 7 est de type Luer mâle, et l'autre embout 8 est de type Luer femelle, pour permettre un emboîtement des embouts de deux plaquettes contiguës.

Dans la réalisation des figures 4 et 5, l'embout mâle 7 comporte un écrou mobile 29 et l'embout femelle 8 comporte une extrémité 10 filetée extérieurement en sorte que l'embout mâle à écrou d'un module puisse être fixé à l'embout femelle fileté du module contigu par vissage de l'écrou sur l'extrémité filetée.

Une plaquette peut porter un robinet ou plusieurs robinets disposés en file, fixé ou fixable par des moyens quelconques appropriés, et peut porter des robinets sur ses deux faces.

Par exemple, la plaquette comporte sur une face une couronne 11 dans laquelle peut être enfoncée l'embase 12 du boisseau 13 du robinet. Le maintien du robinet est assuré par coincement de l'embase ou des embouts. Dans le cas représenté, la couronne 11 comporte des dents 14 entre lesquelles sont coincés les embouts par enfoncement à force.

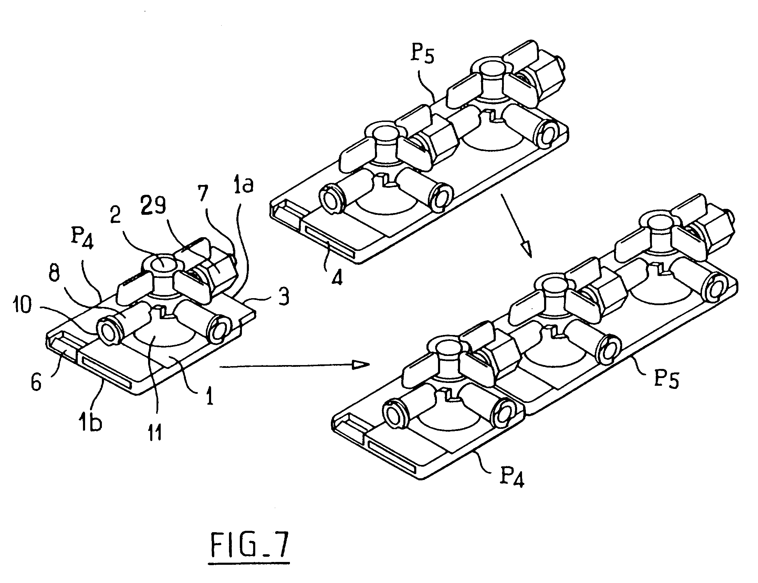

Les figs. 3, 6 et 7 sont des exemples de rampes constituées par assemblage de trois plaquettes identiques (P₁, P₂, P₃ ; P'₁, P'₂, P'₃) (figs. 3 et 6) ou de deux plaquettes différentes (P₄, P₅), l'une P₄ portant un seul robinet et l'autre P₅ portant deux robinets. Il est évident que les exemples ne sont pas limitatifs.

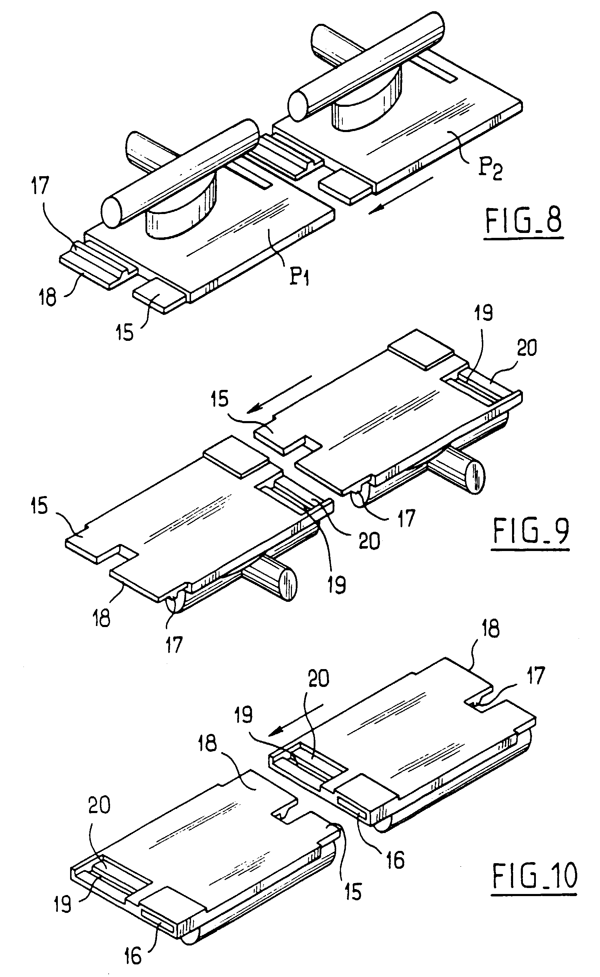

Les figs. 8 à 10 sont relatives à une variante de réalisation dans laquelle l'assemblage de deux plaquettes (P₁, P₂) est obtenu par la coopération, d'une part d'un tenon en saillie 15 sur un bord d'une plaquette et d'un logement 16 en creux dans la tranche de l'autre plaquette et, d'autre part, d'une nervure verticale 17 sur une languette 18 en saillie sur la tranche d'une plaquette et d'une gorge verticale 19 dans une portion d'extrémité 20 de l'autre plaquette pour recevoir ladite nervure.

L'invention n'est pas limitée à ces modes de réalisation.

## Revendications

1. Rampe de robinets, notamment à usage médical, caractérisée en ce qu'elle est constituée de plaquettes et de robinets, chaque plaquette (1) étant munie sur deux bords opposés (1a, 1b) de moyens de solidarisation (3-6) permettant de fixer les plaquettes bout à bout et chaque plaquette portant sur au moins une face un ou plusieurs robinets (2) ou étant munie sur cette face de moyens (11,14) pour le montage d'un ou de plusieurs robinets, et chaque robinet comportant un boisseau (13) dans lequel débouchent au moins trois embouts (7-9) solidaires dudit boisseau , deux des embouts (7-8), respectivement mâle et femelle, étant opposés et alignés selon un axe qui est perpendiculaire aux dits bords opposés de la plaquette lorsque le robinet est fixé sur la plaquette, et le troisième embout (9) étant disposé obliquement ou perpendiculairement par rapport aux deux embouts alignés, ledit robinet étant conçu pour commander une communication de fluide entre les embouts alignés et le troisième embout et lesdits embouts alignés étant conçus pour que ces embouts mâles et femelles s'emboîtent de proche en proche lorsque les plaquettes (1) qui portent les robinets (2) sont solidarisées bout à bout.

2. Rampe selon la revendication 1, caractérisée en ce que les moyens de solidarisation comprennent un tenon (3) sur un bord (1a) de la plaquette et une mortaise (4) sur le bord opposé (1b) de la plaquette.

3. Rampe selon la revendication 1 ou 2, caractérisée en ce que les moyens de solidarisation comprennent deux crampons (5, 6), l'un sur un bord (1a) de la plaquette et l'autre sur le bord opposé (1b) de la plaquette, dans des positions respectives qui permettent au crampon d'un bord d'une plaquette de coopérer avec le crampon opposé du bord d'une plaquette contiguë.

4. Rampe selon la revendication 1 ou 2, caractérisée en ce que les moyens de solidarisation comprennent une nervure verticale (17) sur une languette (18) prolongeant un bord de la plaquette et une gorge verticale (19) dans le bord opposé de la plaquette.

5. Rampe selon l'une des revendications 1 à 4, caractérisée en ce que la plaquette (1) comporte sur une face au moins une couronne (11) pour recevoir l'embase (12) d'un robinet (2).

6. Rampe selon l'une des revendications 1 à 5, caractérisée en ce que les plaquettes (1) sont venues de moulage en résine de synthèse.

7. Rampe selon l'une des revendications 1 à 6, caractérisée en ce que les embouts alignés (8, 7) d'un robinet comportent respectivement une extrémité (10) filetée extérieurement et un écrou mobile (29), en sorte que l'embout à écrou du robinet d'une plaquette puisse être fixé à l'embout fileté du robinet d'une plaquette contiguë par vissage de l'écrou sur l'extrémité filetée.

8. Plaquettes telles que définies dans l'une des revendications 1 à 7.

9. Robinets tels que définis dans l'une des revendications 1 à 7.

## Patentansprüche

1. Plattform mit Hähnen, insbesondere zur medizinischen Verwendung, dadurch gekennzeichnet, daß sie aus Platten und Hähnen besteht, wobei jede Platte (1) an zwei gegenüberliegenden Kanten (1a, 1b) mit Zusammensteckmitteln (3-6) ausgestattet ist, die eine Ende-zu-Ende-Fixierung der Platten ermöglichen, und wobei jede Platte auf wenigstens einer Oberfläche ein oder mehrere Hähne (2) trägt oder auf dieser Oberfläche mit Mitteln (11,14) zur Montage von einem oder mehreren Hähnen ausgestattet ist und jeder Hahn besteht aus einem Hahnküken (13), in das wenigstens drei Ansatzstücke (7-9) einmünden, die mit dem Hahnküken eine Einheit bilden, wobei zwei der Ansatzstücke (7-8), männlich bzw. weiblich, sich gegenüberliegen und an einer Achse ausgerichtet sind, die senkrecht zu den gegenüberliegenden Kanten der Platte ist, wenn der Hahn auf der Platte befestigt ist, und wobei das dritte Ansatzstück (9) schräg oder senkrecht zu den beiden axial ausgerichteten Ansatzstücken angeordnet ist, wobei der Hahn zur Steuerung eines Fluid-Flusses zwischen den axial ausgerichteten Ansatzstücken und dem dritten Ansatzstück ausgelegt ist und die axial ausgerichteten Ansatzstücke ausgelegt sind, damit sich diese männlichen und weiblichen Ansatzstücke nach und nach ineinanderschieben, wenn die Platten (1), die die Hähne (2) tragen, Ende-zu-Ende zusammengesteckt werden.

2. Plattform nach Anspruch 1, dadurch gekennzeichnet, daß die Zusammensteckmittel einen Zapfen (3) auf einer Kante (la) der Platte und ein Zapfenloch (4) auf der gegenüberliegenden Kante der Platte umfassen.

3. Plattform nach Anspruch 1 oder 2, dadurch gekennzeichnet, daß die Zusammensteckmittel zwei Krampen (5,6) umfassen, den einen auf einer Kante (1a) der Platte und den anderen auf der gegenüberliegenden Kante (1b) der Platte in den entsprechenden Positionen, die es dem Krampen von der einen Kante einer Platte ermöglichen, sich mit dem gegenüberliegenden Krampen der Kante einer angrenzenden Platte zu verhaken.

4. Plattform nach Anspruch 1 oder 2, dadurch gekennzeichnet, daß die Zusammensteckmittel eine vertikale Rippe (17) auf einem Fortsatz (18), der eine Kante der Platte verlängert, und eine vertikale Vertiefung (19) in der gegenüberliegenden Kante der Platte umfassen.

5. Plattform nach einem der Ansprüche 1 bis 4, dadurch gekennzeichnet, daß die Platte (1) auf einer Oberfläche wenigstens einen Kranz (11) trägt, um den Fuß (12) eines Hahns (2) aufzunehmen.

6. Plattform nach einem der Ansprüche 1 bis 5, dadurch gekennzeichnet, daß die Platten (1) durch Kunstharzguß hergestellt worden sind.

7. Plattform nach einem der Ansprüche 1 bis 6, dadurch gekennzeichnet, daß die axial ausgerichteten Ansatzstücke (8,7) eines Hahns ein Ende (10) mit Außengewinde und eine bewegliche Mutter (29) aufweisen, damit das Ansatzstück mit Mutter des Hahns der einen Platte an dem Ansatzstück mit Gewinde des Hahns einer angrenzenden Platte durch Aufschrauben der Mutter auf das Ende mit Gewinde befestigt werden kann.

8. Platten nach einem der Ansprüche 1 bis 7.

9. Hähne nach einem der Ansprüche 1 bis 7.

## Claims

1. A strip of cocks, in particular for medical use, characterized in that it is constituted by plates and by cocks, each plate (1) being provided on two opposite edges (1a, 1b) with fastening means (3-6) enabling the plates to be fixed together end-to-end, and each plate carrying on at least one face one or more cocks (2) or being provided on said face with means (11, 14) to which one or more cocks can be mounted, and each cock comprising a plug (13) to which at least three endpieces (7-9) secured to said plug open out, two of the endpieces (7-8), respectively a male endpiece and a female endpiece, being in alignment and opposite to each other on an axis that is perpendicular to said opposite edges of the plate once the cock is fixed on the plate, and the third endpiece (9) being disposed obliquely or perpendicularly relative to the two aligned endpieces, said cock being designed to control fluid communication between the aligned endpieces and the third endpiece, and said aligned endpieces being designed so that said male and female endpieces engage mutually from cock to cock when the plates (1) carrying the cocks (2) are connected together end-to-end.

2. A strip according to claim 1, characterized in that the fastening means comprise a tenon (3) on one edge (la) of the plate and a mortise (4) on the opposite edge (1b) of the plate.

3. A strip according to claim 1 or 2, characterized in that the fastening means comprise two catches (5, 6), one on one edge (la) of the plate and the other on the opposite edge (1b) of the plate, in respective positions that enable the catch on one edge of one plate to co-operate with the opposite catch on the edge of a contiguous plate.

4. A strip according to claim 1 or 2, characterized in that the fastening means comprise a vertical rib (17) on a tongue (18) extending an edge of the plate, and a vertical groove (19) on the opposite edge of the plate.

5. A strip according to any one of claims 1 to 4, characterized in that the plate (1) includes, on a face thereof, at least one ring (11) for receiving the base (12) of a cock (2).

6. A strip according to any one of claims 1 to 5, characterized in that the plates (1) are integrally molded out of a synthetic resin.

7. A strip according to any one of claims 1 to 6, characterized in that the aligned endpieces (8, 7) of a cock respectively include a moving nut (29) and an end (10) having an outside thread, such that the nut-carrying endpiece of the cock on one plate can be fixed to the threaded endpiece of the cock on a contiguous plate by screwing the nut onto the threaded end.

8. A plate as defined in any one of claims 1 to 7.

9. A cock as defined in any one of claims 1 to 7.
